Europäisches Patentamt

European Patent Office

Office européen des brevets

(19)

(11) Publication number: **0 213 751 B1**

# EUROPEAN PATENT SPECIFICATION

(45) Date of publication of patent specification: **28.08.91**   (51) Int. Cl.⁵: **A61M 29/02, A61M 25/00**

(21) Application number: **86305876.4**

(22) Date of filing: **30.07.86**

(54) Steerable dilatation catheter with rotation limiting device.

(30) Priority: **30.07.85 US 760635**

(43) Date of publication of application:
**11.03.87 Bulletin 87/11**

(45) Publication of the grant of the patent:
**28.08.91 Bulletin 91/35**

(84) Designated Contracting States:
**AT CH DE FR GB IT LI NL**

(56) References cited:
**EP-A- 0 165 727**
**FR-A- 965 022**
**US-A- 2 688 882**
**US-A- 3 452 740**

(73) Proprietor: **ADVANCED CARDIOVASCULAR SYSTEMS, INC.**
**1395 Charleston Road**
**Mountain View, CA 94039-7101(US)**

(72) Inventor: **Frisbie, Jeffrey Steven**
**3203 Pine Spring Court**
**San Jose California 95121(US)**
Inventor: **Samson, Wilfred Joseph**
**19691 Falwell Avenue**
**Saratoga California 94070(US)**
Inventor: **Hoek, John Vanden**
**33205 Claremont Street**
**Wildomar California 92395(US)**

(74) Representative: **Bizley, Richard Edward et al**
**BOULT, WADE & TENNANT 27 Furnival Street**
**London EC4A 1PQ(GB)**

## Description

This application relates to a steerable dilatation catheter with a rotation limiting device.

Patent specification US-A-4,582,181 discloses a steerable dilatation catheter. Difficulties have been encountered with this catheter in that surgeons using the same have rotated the guide wire to such an extent that the balloon has been twisted making it difficult to inflate. There is therefore a need for a new and improved steerable dilatation catheter which will overcome this difficulty.

Patent specification FR-A-965022 (Jullien) discloses a device for limiting relative rotation between two parts. In one embodiment, the device comprises a pivot shaft on which is mounted a screw-threaded shaft, a plurality of washers and a stationary part. The stationary part and washers have projections which engage each other after less than one rotation and so limit rotation of the stationary part.

In general, a low profile steerable dilatation catheter assembly comprises a guide wire having proximal and distal ends. An adapter is provided with at least one arm having the guide wire extending therethrough. An elongate flexible tubular member extends over the guide wire from the adapter to a region near the distal extremity of the guide wire. A flexible tip is carried by the guide wire. The tubular member carries an inflatable balloon that has its distal extremity secured to the core wire so as to provide a liquid-tight seal.

The present invention provides in one aspect a steerable dilatation catheter comprising an adapter having an arm, a guide wire and a rotation limiting device attached to the arm of the adapter, the guide wire extending through the arm and being secured to the device, characterised in that the device comprises a rotatable member or housing, an adapter and a plurality of discs, all arranged on a common axis and rotatable with respect to one another, the adapter at one end of the device being secured to the arm, the member being in the form of a cylindrical cap with, at the other end of the device, an end wall and a side surrounding the discs and at least a part of the adapter, the guide wire being secured to the member, the adapter having an arcuate guideway in the form of a recess, the member having an internal protrusion, and the discs having arcuate guideways in the form of recesses and having protrusions, each protrusion engaging in a respective recess for relative and limited rotation of the member with respect to the adapter.

In another aspect the invention provides a guidewire and a rotation limiting device on the proximal end of the guide wire, comprising: a rotatable housing having means to secure thereto the proximal end of the guide wire; a plurality of discs disposed adjacent to one another in axial alignment and adapted to rotate therein, each disc having an arcuate guideway in the form of a recess adapted to be followed by a protrusion of an adjacent disc; characterised in that the discs are disposed in a cylindrical recess of the housing; in that the housing has an internal protrusion engaging the arcuate recess of the closest disc in the recess; and in that the device comprises an adapter engaged with and freely rotatable with respect to the housing and having an arcuate guideway in the form of a recess adapted to be followed by the protrusion of the closest disc in the recess.

In the accompanying drawings,

Figure 1 is a partial side elevational view of a catheter assembly with a rotation limiting device;

Figure 2 is a cross sectional view of the rotation limiting device shown in Figure 1; and

Figure 3 is an exploded view of the rotation limiting device shown in Figure 2.

The steerable dilatation catheter assembly with a rotation limiting device as shown in the drawings comprises an adapter 12 having a central arm 16 and two side arms 17 and 18. A rotation limiting device 92 is mounted on the central arm 16 of the adapter 12. The rotation limiting device 92 includes a rotatable member 93 which is in the form of a cylindrical cap which is provided with a side wall 94 and a top or end wall 96. The side wall 94 is provided with circumferentially spaced outwardly facing recesses 97 extending longitudinally thereof which facilitate gripping of the outer surface by hand during operation as hereinafter described. The top wall 96 is provided with a centrally disposed hole 98 which opens into a recess 99. The hole 98 also opens into a centrally disposed bore 101 which extends through a centrally disposed cylindrical stem 102 concentric with the side wall 94. A cylindrical recess 103 is provided within the rotatable member 93.

The cap or housing which is formed by the rotatable member 93 is rotatably mounted upon an adapter 104 which is adapted to be mounted on the central arm 16 by suitable means such as an adhesive fit. The adapter 104 is provided with a truncated conical surface 106 which facilitates this mounting. The adapter is provided with a centrally disposed bore 107 which is adapted to receive the distal extremity of the stem 102. The adapter 104 is provided with an annular recess 108 which is adapted to carry suitable sealing means such as an O-ring 109. The O-ring 109 thus serves to form a liquid-tight seal between the adapter 104 and the stem 102 of the housing 93.

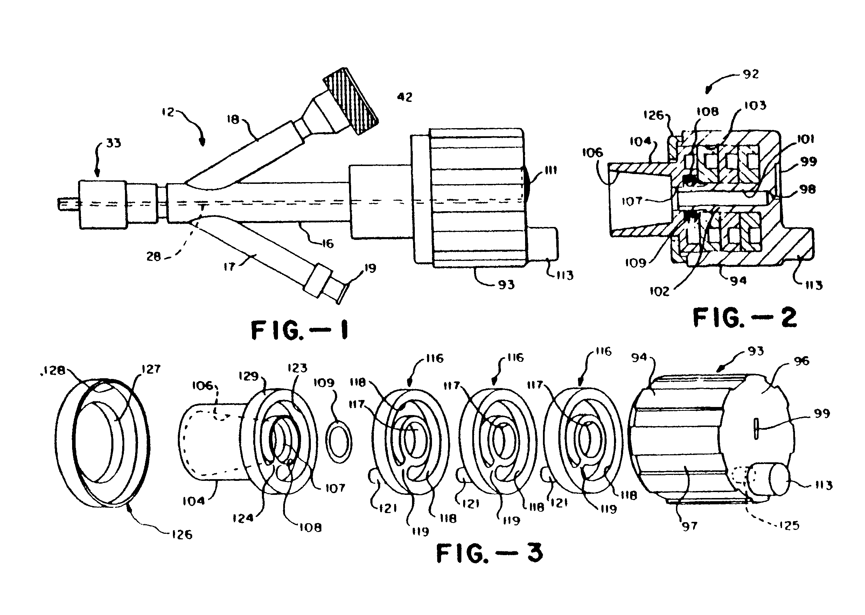

Referring in particular to Figure 1, a guide wire 28 extends through the bore 101 and in the hole 98 and is bent into the recess 99. A suitable sealing means, such as an epoxy 111, secures the end of the guide wire 28 in the recess 99. A protrusion 113 is provided on the top wall 96 and extends outwardly therefrom. It is adapted to be engaged by a finger of a hand to facilitate rotation of the rotatable member 93. The proximal extremity of the guide wire 28, since it is secured to the rotatable member, will be rotated with the rotatable member.

Means is provided in the rotation limiting device 92 for limiting the rotation to no more than a predetermined number of turns as, for example, four complete turns or revolutions. This means comprises a plurality of discs 116 which are disposed within the recess 103 as, for example, the three discs shown in Figure 2. The discs 116 are substantially identical. Each of the discs 116 is provided with a central bore 117 and a arcuate recess 118 which is generally concentric with the bore 117. The arcuate recess 118 extends through substantially 360° except for a small web 119. Each disc has an axially extending cylindrical protrusion 121 which is adapted to seat in and travel in the arcuate recess 118 provided in the adjacent disc. The protrusion 121 of the extreme left hand disc (see Figure 3) is adapted to seat in an arcuate recess 123 provided in the adapter 104. The recess 123 is similar to the recesses 118 and extends through substantially 360° except for a small web 124. The rotatable member 93 is provided with a protrusion 125 extending inwardly from the top wall 96 and which is adapted to seat in the arcuate recess 118 of the extreme right hand disc 116.

In assembling the rotation limiting device, the discs are inserted into the housing-like rotatable member 93 so that the first disc 116 has the protrusion 125 seated in its recess 118 and so that the protrusions 121 of each of the discs are disposed in the recesses 118 of the adjacent discs. A retainer 126 is provided for retaining the adapter 104 within the open end of the housing-like rotatable member 93 and is provided with a central opening 127 and an annular outer rim 128. The retainer 126 is adapted to engage but rotate freely with respect to a shoulder 129 of the adapter 104 and to have the rim 128 bonded to the outer extremity of the housing-like rotatable member 93 by suitable means such as an adhesive. When assembled in this manner, the various parts of the rotation device are held together in a unitary assembly while still permitting rotation of the housing 93 relative to the adapter 104 through a predetermined number of revolutions as, for example, approximately four. The number of revolutions is determined by the number of discs 116 and thus by the number of arcuate slots 118 which are provided. As can be seen there are three slots 118 provided in the three discs 116 and another arcuate slot 123 is provided in the adapter 104.

Thus it can be seen that there has been provided a catheter assembly with a rotation limiting device which has an attractive appearance which is relatively simple to fabricate and assemble. It also provides for the maximum desired number of rotations for the guide wire.

It is apparent from the foregoing that there has been provided a low profile steerable dilatation catheter of the type which limits twisting movement of the balloon while at the same time providing sufficient torsional rigidity so that there is at least 360° rotation of the tip of the dilatation catheter and therefore it does not detract from the capabilities of the dilatation catheter. The additional torsional rigidity required in the catheter assembly with no more than two turns of the torque knob can be readily accomplished. The dilatation catheter is also of the type which can be readily manufactured and assembled.

## Claims

1. A steerable dilatation catheter comprising an adapter (12) having an arm (16), a guide wire (28) and a rotation limiting device (92) attached to the arm (16) of the adapter (12), the guide wire (28) extending through the arm (16) and being secured to the device (92), characterised in that the device (92) comprises a rotatable member or housing (93), an adapter (104) and a plurality of discs (116), all arranged on a common axis and rotatable with respect to one another, the adapter (104) at one end of the device being secured to the arm (16), the member (93) being in the form of a cylindrical cap with, at the other end of the device, an end wall (96) and a side (94) surrounding the discs (116) and at least a part of the adapter (104), the guide wire (28) being secured to the member (93), the adapter (104) having an arcuate guideway (123) in the form of a recess, the member (93) having an internal protrusion (125), and the discs (116) having arcuate guideways (118) in the form of recesses and having protrusions (121), each protrusion (125, 121) engaging in a respective recess (123, 118) for relative and limited rotation of the member (93) with respect to the adapter (104).

2. A guidewire and a rotation limiting device on the proximal end of the guidewire, comprising:
   a rotatable housing (93) having means (98,99) to secure thereto the proximal end of the guide wire (28);
   a plurality of discs (116) disposed adjacent to one another in axial alignment and adapted to rotate therein, each disc (116) having an arcuate guideway in the form of a recess (118) adapted to be followed by a protrusion (121) of an adjacent disc;

characterised in that the discs (116) are disposed in a cylindrical recess (103) of the housing (93);

in that the housing (93) has an internal protrusion (125) engaging the arcuate recess (118) of the closest disc (116) in the recess (103); and

in that the device comprises an adapter (104) engaged with and freely rotatable with respect to the housing (93) and having an arcuate guideway in the form of a recess (123) adapted to be followed by the protrusion of the closest disc (116) in the recess (103).

3. A device as claimed in claim 2, wherein the housing (93) is provided with a stem (102) about which the discs (116) are rotatable mounted.

4. A device as claimed in claim 3, wherein each guideway recess (118, 123) has a web (119, 124) limiting movement of the respective protrusion (121,125) in the guideway recess (118, 123).

**Revendications**

1. Dilatateur dirigeable comprenant un adaptateur (12) composé d'un bras (16), d'un fil de guidage (28) et d'un dispositif limitateur de rotation (92) fixé au bras (16) de l'adaptateur (12), le fil de guidage (28) s'étendant à travers le bras (16) et étant fixé au dispositif (92), caractérisé en ce que le dispositif (92) comprend un élément (93) ou logement rotatif, un adaptateur (104) et une pluralité de disques (116), tous ces éléments étant disposés sur un axe commun et pouvant tourner les uns par rapport aux autres, l'adaptateur (104) étant fixé au bras (16) à une extrémité du dispositif, l'élément (93) ayant la forme d'un couvercle cylindrique avec, à l'autre extrémité du dispositif, une paroi terminale (96) et un côté (94) entourant les disques (116) et au moins une partie de l'adaptateur (104), le fil de guidage (28) étant fixé à l'élément (93), l'adaptateur (104) ayant un passage de guidage incurvé (123) en forme de creux, l'élément (93) ayant une avancée intérieure (125), et les disques (116) ayant des passages de guidage incurvés (118) sous forme d'évidements et présentant des avancées (121), chaque avancée (125, 121) s'engageant dans un évidement correspondant (123, 118) pour une rotation relative et limitée de l'élément (93) par rapport à l'adaptateur (104).

2. Fil de guidage et dispositif limitateur de rotation sur l'extrémité proximale du fil de guidage, comprenant un logement rotatif (93) ayant des moyens (98, 99) pour y fixer l'extrémité proximale du fil de guidage (28), une pluralité de disques (116) disposés côte à côte en alignement axial et adaptés pour tourner dans ce logement, chaque disque (116) ayant un passage de guidage incurvé sous forme d'un évidement (118) pouvant être suivi par une avancée (121) d'un disque adjacent, caractérisé en ce que les disques (116) sont disposés dans un évidement cylindrique (103) du logement (93), en ce que le logement (93) possède une avancée intérieure (125) s'engageant dans l'évidement incurvé (118) du disque le plus proche (116) dans l'évidement (103), et en ce que le dispositif comprend un adaptateur (104) en prise dans le logement (93) et pouvant tourner librement, et ayant un conduit de guidage incurvé sous forme d'un évidement (123) conçu pour être suivi par une avancée du disque le plus proche (116) dans l'évidement (107).

3. Dispositif selon la revendication 2, caractérisé en ce que le logement (93) est pourvu d'une tige (102) autour de laquelle les disques (116) sont montés en rotation.

4. Dispositif selon la revendication 3, caractérisé en ce que chaque évidement de passage de guidage (118, 123) a une nervure (119, 124) limitant le mouvement de l'avancée respective (121, 125) dans l'évidement de passage de guidage (118, 123).

**Patentansprüche**

1. Steuerbarer Dilatationskatheter umfassend einen Adapter (12) mit einem Arm (16), eine an dem Arm (16) angebrachte Rotationsbegrenzungsvorrichtung (92) und einen sich durch den Arm (16) hindurch erstreckenden Führungsdraht (28), der an der Rotationsbegrenzungsvorrichtung (92) befestigt ist,
**dadurch gekennzeichnet,**
daß die Rotationsbegrenzungsvorrichtung (92) ein drehbares Element oder Gehäuse (93), einen Adapter (104) und eine Vielzahl von auf einer gemeinsamen Achse angeordneten und relativ zueinander drehbaren Scheiben (116) umfaßt, daß an einem Ende der Rotationsbegrenzungsvorrichtung (92) der Adapter (104) an dem Arm (16) befestigt ist, daß das Element (93) die Form einer zylindrischen Kappe mit einer Abschlußwand (96) an dem anderen Ende der Rotationsbegrenzungsvorrichtung (92) und einer die Scheiben (116) und wenigstens einen Teil des Adapters (104) umgebenden Seitenwand (94) hat, daß der Führungsdraht (28) an dem Element (93) befestigt ist, daß der Adapter (104) eine bogenförmige Führung (123) in Form einer Aussparung aufweist, daß das Element (93) einen internen Vorsprung (125) hat und daß die Scheiben (116) Vorsprünge (121) und bogenförmige Führungen (118) in Form von Aussparungen aufweisen. wobei jeder Vorsprung (125, 121) in eine jeweilige Aussparung (123, 118) eingreift, um eine begrenzte Drehung des Elementes (93) relativ zu dem Adapter (104) vorzusehen,

2. Führungsdraht und Rotationsbegrenzungsvorrichtung an dem proximalen Ende des Führungsdrahtes, umfassend:

ein drehbares Gehäuse (93), das Mittel (98, 99) zur Befestigung des proximalen Endes des Führungsdrahtes (28) an dem Gehäuse (93) aufweist,

eine Vielzahl von einander benachbarten und axial zueinander ausgerichteten drehbaren Scheiben (116), von denen jede eine bogenförmige Führung in Form einer Aussparung (118) aufweist, die dazu vorgesehen ist, daß ihr ein Vorsprung (121) einer benachbarten Scheibe (116) folgt,

**dadurch gekennzeichnet,**

daß die Scheiben (116) in einer zylindrischen Aussparung (103) des Gehäuses (93) angeordnet sind,

daß das Gehäuse (93) einen internen Vorsprung (125) aufweist, der in die bogenförmige Aussparung (118) der nächstbenachbarten Scheibe (116) in der zylindrischen Aussparung (103) eingreift,

und daß die Rotationsbegrenzungsvorrichtung einen mit dem Gehäuse (93) in Eingriff stehenden und relativ zu dem Gehäuse (93) frei drehbaren Adapter (104) aufweist, der eine bogenförmige Führung in Form einer Aussparung (123) hat, die dazu vorgesehen ist, daß ihr ein Vorsprung der nächstbenachbarten Scheibe (116) in der zylindrischen Aussparung (103) folgt.

3. Vorrichtung nach Anspruch 2,
   **dadurch gekennzeichnet,**
   daß das Gehäuse (93) mit einem Schaft (102) versehen ist, auf dem die Scheiben (116) drehbar gelagert sind.

4. Vorrichtung nach Anspruch 3,
   **dadurch gekennzeichnet,**

daß jede Führungsaussparung (118, 123) einen Steg (119, 124) aufweist, der die Bewegung des jeweiligen Vorsprungs (121, 125) in der Führungsaussparung begrenzt.

FIG.—1

FIG.—2

FIG.—3